(19) Europäisches Patentamt
European Patent Office
Office européen des brevets

(11) **EP 3 648 843 B1**

(12) **EUROPEAN PATENT SPECIFICATION**

(45) Date of publication and mention
of the grant of the patent:
**07.09.2022 Bulletin 2022/36**

(21) Application number: **18736887.3**

(22) Date of filing: **02.07.2018**

(51) International Patent Classification (IPC):
*A61Q 15/00* (2006.01)    *A61K 8/73* (2006.01)
*A61K 8/26* (2006.01)    *A61K 8/20* (2006.01)

(52) Cooperative Patent Classification (CPC):
**A61Q 15/00; A61K 8/20; A61K 8/26; A61K 8/731**

(86) International application number:
**PCT/EP2018/067846**

(87) International publication number:
**WO 2019/007916 (10.01.2019 Gazette 2019/02)**

(54) **COMPOSITION COMPRISING ANTIPERSPIRANT ACTIVE AND MICROFIBRILS**

ZUSAMMENSETZUNG MIT SCHWEISSHEMMENDEM WIRKSTOFF UND MIKROFIBRILLEN

COMPOSITION COMPRENANT UN ACTIF ANTI-TRANSPIRANT ET DES MICROFIBRILLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.07.2017 EP 17179614**

(43) Date of publication of application:
**13.05.2020 Bulletin 2020/20**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU
  IS LI LT LU LV MC MK NL NO PL PT RO RS SE SI
  SK SM TR**
- **Unilever Global IP Limited**
  **Wirral, Merseyside CH62 4ZD (GB)**
  Designated Contracting States:
  **CY DE GB IE IT MT**

(72) Inventors:
- **VERHEIJ, Jan Adrianus**
  **3119 VJ Schiedam (NL)**
- **VELIKOV, Krassimir Petkov**
  **3133 AT Vlaardingen (NL)**

(74) Representative: **Whaley, Christopher
Unilever N.V.
Unilever Patent Group
Bronland 14
6708 WH Wageningen (NL)**

(56) References cited:
**WO-A1-00/21498        WO-A1-2005/018594
WO-A1-2017/067899    US-A1- 2014 363 560**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

As illustrated in the appended claims, the present disclosure relates to an aqueous antiperspirant composition comprising a water soluble antiperspirant active and a defibrillated primary cell wall material sourced from plant parenchymal tissue, to a process for manufacture of said composition, to a non-therapeutic topical application of said composition to human skin and to a method of thickening the aqueous antiperspirant composition.

**BACKGROUND TO THE INVENTION**

Antiperspirant composition traditionally comprises astringent salt and particularly a basic salt such as a basic aluminium salt. The aluminium salt comprising chloride anion is water soluble, producing isotropic, Newtonian solution with clarity. The Newtonian solution can be characterized by its rheology wherein its viscosity does not depend upon the shear rate and it has the ratio of elastic modulus G' to viscous modulus G" (G'/G") smaller than 1. However, the majority of antiperspirant products (such as deodorant gels, creams, roll-ons and lotions) are structured liquid or soft solid, wherein G'/G" ≥1 and shear thinning property are required to achieve the desirable rheological profile in use. This is because a Newtonian solution (G'/G"<1) is very thin and may easily drip from its dispenser, in particular, from traditional roll-on applicators comprising a roll-ball. This can result in consumers having difficulty to control the dosage amount and/or the dispenser becoming messy. Overdosing can leave great wetness in consumers' underarm areas. Further, a thin composition may drip from the underarm after application, which undesirably wet other parts of the skin as well as clothes.

The difficulty *vide supra* can be worsened by the composition further comprising a co-solute and/or a dispersed phase that is solid, immiscible liquid (e.g. oils), gas or mixtures thereof. The composition may suffer from stability problem that relates to phase changes such as sedimentation and creaming.

Accordingly, the dripping problem makes the product less appealing to consumers. Therefore there is a need to increase G' value of the antiperspirant active solution without aesthetically affecting its appearance and still allowing the product to be easily applied on skin.

Many synthetic polymers have been used in antiperspirant composition to solve the rheology problem. However, consumers are increasingly desirous of applying only 'natural' ingredients and treatments to their body. Synthetic ingredients do not satisfy this demand for 'natural' origin.

It is also known that the use of high level of antiperspirant active is commonplace to achieve enhanced, long-lasting efficacy. Many compositions contain at least 2% aluminium chlorohydrate salt by weight. The high concentration of $Al^{3+}$ and $Cl^-$ ions gives high ionic strength $I_c$ $(I_c = 0.5 \sum_i z_i^2 c_i$ where $z_i$ and $c_i$ represent the charge number and the concentration of species i, respectively). For example, the ionic strength of 2% aluminium chlorohydrate salt is typically at least 0.5M. Such high ionic strength leaves the composition difficult to thicken by natural ingredients. It is because the swelling behavior of natural plant cell is largely suppressed by the high ionic strength and it is inefficient in space filling. M.A Joslyn and Guido De Luca, J.Colloid Sci., 12, 1, 108-130 (1957) describes the precipitation of aluminium pectinates from pectin. Many biopolymers are also not effective because their solubility is largely affected by the high ionic strength as well.

Defibrillated cell wall materials have been used in personal and household industry. WO12084441 and WO12084421 describes the use of microfibrillated cellulose as structurant in non-aqueous compositions. WO14082835 describes the use of microfibrillated cellulose fibres in aqueous cleansing composition for hard surface. The fibre is said to suspend calcite particles as well as increase cleansing power. WO14082951 describes the use of microfibrillated cellulose fibres in toothpaste comprising calcite particles as well. WO15006635, WO15006634, WO13160022, WO12052306, US2015210957 and WO13160024 describe the use of cellulose fibres in laundry products. The fibres are used to structure detergent or fabric conditioners with very low level of divalent cations (such as $Ca^{2+}$ and $Mg^{2+}$). US2012040003 and WO13107771 describe the use of citrus fibres in shampoo composition. The former only concerns with delivering anti-dandruff zinc salt actives in a cleansing medium that maintains phase stability, holds appropriate viscosity and enhances deposition of actives. The latter mitigates the problem of instability, in particular phase separation, sedimentation and rancid odour of coloured compositions. To date, there is no teaching of using cellulose fibres in antiperspirant products to control dripping and associated problems.

**SUMMARY OF THE INVENTION**

As essential aspect of the present invention, there is provided an aqueous antiperspirant composition comprising:i) at least 2 wt% of water soluble, antiperspirant active comprising aluminium ions and chloride ions, wherein the

EP 3 648 843 B1

water soluble antiperspirant active is an astringent antiperspirant salt selected from an aluminium or an aluminium-zirconium chlorohydrate salt or complex, and ii) from 0.1 to 4.0 wt% of defibrillated primary cell wall material, wherein at least 50 wt% of the total material is sourced from plant parenchymal tissue and wherein the defibrillated primary cell wall material comprises at least 50 wt% of cellulose microfibrils, based upon the total dry weight of the defibrillated primary cell wall material.

In a second aspect of the present invention, there is provided an aqueous antiperspirant composition according to the first aspect, in the form of a roll-on, lotion, cream or gel, preferably a roll-on, contained within a dispenser that allows flow of the composition onto the surface of the human body.

In a third aspect of the present invention, there is provided a method of manufacturing an antiperspirant composition according to the first aspect, comprising defibrillation in an aqueous medium of the primary cell wall material wherein during or after the defibrillation, a water soluble antiperspirant active is mixed in.

In a fourth aspect of the present invention, there is provided a method of reducing perspiration from human skin comprising the topical application of the composition according to the first aspect of the invention. The said method typically involves applying an effective amount of the composition to the human skin, in particular to the underarm areas, otherwise known as the axillae.

In a fifth aspect of the present invention, there is provided a method of thickening an aqueous antiperspirant composition comprising at least 2 wt.% of water soluble antiperspirant active comprising aluminium ions and chloride ions, said method comprising the addition of from 0.1 to 4.0 wt.% of defibrillated primary cell wall material sourced from plant parenchymal tissue.

An objective of the present invention is to provide a natural additive that can reduce dripping problem of an aqueous antiperspirant product comprising high level of antiperspirant active, e.g. at least 2 wt%. Related benefits of the natural additive may also include reduced wet feel, improvement of stability and dispensing. In addition, the additive should not affect the appearance of the composition adversely.

Surprisingly, it has been found that the inclusion of defibrillated primary cell wall material into an aqueous antiperspirant composition with high ionic strength gives the composition higher elastic modulus than viscous modulus (G'/G">1), thus reducing the dripping problem. The composition is also stable upon transport and storage both at ambient and elevated temperature. It has also been found the composition can maintain its aesthetic clarity.

## DETAILED DESCRIPTION OF THE INVENTION

Any feature of a particular embodiment of the present invention may be utilized in any other embodiment of the invention. The word 'comprising' is intended to mean 'including' but not necessarily 'consisting of' or 'composed of'. The examples given in the description below are intended to clarify the invention but not to limit the invention. All weight percentages (wt%) are based upon the final weight of the composition unless indicated otherwise. Numerical ranges expressed in the format 'x-y' are understood to include x and y, unless specified otherwise. The numbers can be qualified by the term 'about'.

The aqueous antiperspirant compositions of the invention comprise preferably at least 40 wt% of water, more preferably at least 60 wt% of water, even more preferably at least 70 wt% of water.

*Antiperspirant active*

The antiperspirant composition comprises at least 2 wt% of water soluble antiperspirant active, more preferably 4-25 wt%, even more preferably 10-20 wt% Herein the term 'water soluble' should be understood to mean the antiperspirant active has a solubility in demineralised water of greater than 0.1g/100mL at 20°C.

The antiperspirant active for use herein is selected from aluminium, aluminium/ziconium and mixture thereof, including both inorganic salts, salts with organic anions and complexes. The anion of the antiperspirant active comprises chloride. The preferred actives include aluminium, aluminium/ziconium chloride and chlorohydrate salts, such as aluminium chloride and aluminium chlorohydrate. The especailly preferred salts are basic salts, which is to say a fraction of the chloride within the empirical formula has been replaced by bound hydroxyl groups. Chlorohydrate salts are very highly desired. Aluminium chlorohydrate has a general fomula $Al_2(OH)_xCl_y \cdot wH_2O$, wherein x is variable from 2 to 5; x+y=6 wherein x and y are not necessarily integers; $wH_2O$ represents a variable amount of hydration. Aluminium chlorohydrates are made comprises a mixture of a number of different polymeric species in varying proportions, depending on the molar ratio of aluminium to chloride and the conditions employed during manufacture. All such mixtures are employable herein. It is especially desirable to employ what is commonly called activated aluminium chlorohydrate or enhanced activity aluminium chlorohydrate, sometimes abbreviated to AACH, in which the proportion of the more active species, such as Band III species determined by SEC (Size Exclusion Chromatography) is higher by virtue of its method of manufacture. The exemplary AACH is described in EP0006739. AACH is also commercially available by name, or as actrivated or enhanced activity, from suppliers such as Reheis, Summit Research and B K Giulini.

3

A particularly preferred abtiperspirant active is aluminium sesquichlorohydrate (herein ASCH) of chemical formula $Al_2OH_{4.4}Cl_{1.6}$ to $Al_2OH_{4.9}C_{1.1}$. and particularly of chemical formula $Al_2OH_{4.7}Cl_{1.3}$ to $Al_2OH_{4.9}Cl_{1.1}$. It is further preferred that such an ASCH salt is activated with calcium and optionally glycine as described in WO 2014/187685, for example.The above aluminium salts may have coordinated and /or bound water in various quantities and/or may be present as polymeric species, mixtures or complexes. In particular, aluminium zirconium chlorohydrate is preferred.

The antiperpirant complexes based on the aforementioned aluminium and/or aluminium/zirconium salts can also be exmployed. The complex often employs a compound with a carboxylate group, and advantageously this is an amino acid. The exemplary amino acids include dl-tryptophan, dl-β-phenylalanine, dl-valine, dl-methionine , β-alanine and preferably glycine. It is highly desirable to employ a complexes of combination of aluminium chlorohydrate, aluminium/ziconium chlorohydrate together with amino acids such as glycine, which as disclosed in US3792068. Actives of this type are available from Westwood, from Summit and from Reheis.

The percentage of antiperspirant active in composition normally includes the weight of any hydration and any complexing agent that may present in the active.

*Defibrillated primary cell wall material*

In general, primary cell wall material typically contains cellulose microfibrils, hemicellulose, pectin and in many cases lignin. The primary cell wall material according to present invention may comprise lignin only in minor amount, more preferably at most 10 wt% calculated on total amount of the cell wall material and most preferably does not comprise lignified tissue.

At least 50 wt%, preferably at least 75 wt% and more preferably at least 90 wt% of the primary cell wall material is derived from plant parenchymal tissue. The source of the plant parenchyma cells may be any plant that contains plant parenchyma cells having a cellulose skeleton.

The parenchymal tissue of the primary cell wall material is preferably derived from fruits, roots, bulbs, tubers, seeds, leaves and mixtures thereof; more preferably is derived from citrus fruit, tomato fruit, peach fruit, pumpkin fruit, kiwi fruit, apple fruit, mango fruit, sugar cane, beet root, turnip, parsnip, maize, oat, wheat, peas and mixture thereof; even more preferably is derived from citrus fruit, tomato fruit and sugar cane. The most preferred source is from citrus fruit.

The primary cell wall material preferably has undergone several pre-treatment steps before it is defibrillated. Such pre-treatments preferably comprise one or more steps of heating, cooking, washing, refining, depectinating; and more preferably comprise steps of washing and/or depectinating. Preferably the 'primary cell wall material' is primary cell wall material from which most (*i.e.* more than 50 wt%) of all water-soluble components, and preferably essentially all, water soluble components have been removed. Water-soluble components are those removable by washing with water at temperature of 20°C. The dry weight of primary cell wall material, corresponding to the insoluble fibre fraction, is determined by the following protocol. The dispersion containing primary cell wall material is centrifuged for 60 minutes at 4250 relative centrifugal force (RCF). The obtained pellets are re-dispersed into 800g dispersion (total weight) using demineralized water. The dispersion is centrifuged for 30 minutes at 4250 RCF. The obtained pellets are re-dispersed into 800g dispersion (total weight) and centrifuged again for 20 minutes at 4250 RCF. The dry weight of the obtained pellet is measured using an infrared oven scale (Mettler Toledo HB43-S) at 120°C.

The primary cell wall material of present invention need not contain hemicellulose and pectin. Preferably the primary cell wall material comprises at most 40 wt%, more preferably at most 30 wt%, even more preferably at most 20 wt% and most preferably at most 5 wt% hemicellulose, based upon the total dry weight of the cell wall material. Likewise, the primary cell wall material preferably comprises at most 30 wt%, more preferably at most 25 wt%, even more preferably at most 20 wt% and most preferably at most 5 wt% pectin. The hemicellulose and pectin could be removed during the pre-treatment steps.

The antiperspirant composition according to present invention, comprises 0.1-4.0 wt%, more preferably 0.1-3 wt%, even more preferably 0.3-2.5% and most preferably 0.5-1.0 wt% defibrillated primary cell wall material, wherein essentially all water soluble components have been removed.

Cellulose microfibrils are well-known in the art. A typical microfibril comprises 20-50 aligned beta-1-4-glucose polymer chains. The primary cell wall material according to the invention comprises preferably at least 50 wt%, more preferably at least 60 wt%, even more preferably at least 70 wt%, still more preferably at least 80 wt% and still even more preferably at least 90 wt% of cellulose microfibrils, based upon total dry weight of the primary cell wall material. Most preferably, the primary cell wall material consists essentially of cellulose microfibrils.

The weight percentage of cellulose microfibrils in the primary cell wall material preferably is increased by removing soluble and unbound sugars, protein, polysaccharides, oil soluble oils, waxes and phytochemicals (e.g. carotenoids, lycopene). This is suitably achieved using well-known techniques to the skilled person, including cutting up the cell wall material, cooking, washing, centrifugation, decanting and drying.

The primary cell wall material according to the invention preferably has an average degree of crystallinity of less than 50%. More preferably the average degree of crystallinity is at most 40%, still more preferably at most 35% and most preferably at most 30%. Table 1 shows the average degree of crystallinity of typical sources of cellulose microfibrils. It shows that the cellulose in primary cell wall material sourced from plant parenchymal tissue typically has a degree of crystallinity of less than 50 wt%.

**Table 1:** Average degree of crystallinity of cellulose (all polymorph cellulose I).

| Source | Average degree of crystallinity (%) |
|---|---|
| Tomato fibers | 32 |
| Citrus fiber (Citrus Fiber AQ+N) | 29 |
| Nata de Coco | 74 |
| Cotton | 72 |
| Wood pulp fiber (Meadwestvaco) | 61 |
| Sugar beet fibre (Nordix Fibrex) | 21 |
| Pea fibres (PF200vitacel) | 42 |
| Oat fibres (780 Sunopta) | 43 |
| Corn hull (Z-trim) | 48 |
| Sugar cane Fiber (Ultracel) | 49 |

The average degree of crystallinity is measured according to the following method using wide angle X-ray scattering (WAXS). The measurements are performed on a Bruker D8 Discover X-ray diffractometer with GADDS (General Area Detector Diffraction System) (From Bruker-AXS, Delft, NL) (Part No: 882-014900 Serial No: 02-826) in a theta/theta configuration. A copper anode is used, and the K- alpha radiation with wavelength 0.15418 nm is selected. The used instrumental parameters are shown in table 2.

**Table 2:** D8 Discover instrumental parameters for WAXS measurements.

| | 2θ (9 - 42°) |
|---|---|
| Theta 1 | 10.000 |
| Theta 2 | 10.000 / 25.000 |
| Detector Bias (kV / mA) | 40 / 40 |
| Time (sec) | 300 |
| Collimator (mm) | 0.3 |
| Detector distance (cm) | 25 |
| Tube Anode | Cu |

The average degree of crystallinity (Xc) is calculated from the following equation:

$$Xc\ (\%) = \frac{\text{Area crystalline phase}}{\text{Area crystalline} + \text{amorphous phase}} * 100\%$$

The areas of the diffraction lines of the crystalline phase are separated from the area of the amorphous phase by using the Bruker EVA software (version 12.0).

Cellulose microfibrils are strongly self-associated in the cell walls or fibres. Secondary cell walls, which are mainly found in wood, are distinct from primary cell walls. In the secondary cell walls, microfibrils are organized in the form of highly oriented sheets thus forming an in-dissociable fibre. They are conventionally in the form of aggregates from a few tens of nanometres to a few micrometres. These aggregates consist of elementary microfibrils which may not be easily disentangled during the homogenization without breaking.

The primary cell wall material according to present invention comprises defibrillated cell wall material, *i.e.* the cellulose microfibrils that are present in the primary cell wall are at least partially disentangled, preferably without substantially breaking them. The primary cell wall material can be defibrillated by mechanical energy and/or cavitation thereby disentangling the cellulose microfibrils. Alternatively, the primary cell wall material can be combined with one or more of the ingredients of antiperspirant composition wherein the mixture is subjected to mechanical energy and/or cavitation thereby disentangling the cellulose microfibrils. Preferably the average length of the cellulose microfibrils in the primary cell wall material is more than 1 micrometre and more preferably more than 5 micrometres. The diameter and crystallinity of the fibres do not alter after defibrillated process.

Preferably at least 80 wt% of the primary cell wall materials are smaller than 50nm in diameter, more preferably at least 80 wt% of the primary cell wall materials are smaller than 40 nm in diameter, still more preferably at least 80 wt% of the primary cell wall materials are smaller than 30nm, even more preferably at least 80 wt% of the primary cell wall materials are smaller than 20nm and most preferably are smaller than 10nm. The diameter is determined using transmission electron microscopy (TEM) according to E. Harris et, al. Tools for cellulose analysis in plant cell walls plant physiology, 2010(153), 420. In particular, a dispersion of plant source rich in primary cell wall material is diluted in distilled water resulting in a thin layer. These dispersions are then imaged on a carbon only 300 mesh Copper TEM grid (Agar Scientific) using a Tecnai 20 Transmission electron microscope (FEI Company) operated at a voltage of 200 kV. To enhance image contrast between individual microfibrils, a 2% phosphotungstic acid solution at pH 5.2 is used as a negative stain. To do this the fibre-loaded TEM grids are incubated with 2% phosphotungstic acid and air-dried after removal of the excess of fluid. US2001/0004869A1 sets forth the diameter and crystallinity of the cellulose microfibrils despite the use of word 'nanofibrils' rather than 'microfibrils'.

Polymers

The invention may further comprise an additional additive to aid in obtaining the desired viscosity and suppress syneresis. The additive can be hydrocolloids which may include hydrocolloids selected from gums, polysaccharides, gelatin and glycoproteins. Preferably, such hydrocolloid is selected from agar, agarose, pectin, hemicelluloses, modified celluloses, microcrystalline cellulose, sodium alginate, guar, beta-glucan, tara gum, carrageenan, gum Arabic, locust bean gum, gelatin, starch, modified starch, xanthan gum and mixtures thereof.

Additional hydrocolloids may comprise from 0.005 to 1 wt%, more preferably from 0.01 to 0.8 wt%, even more preferably from 0.05 to 0.5 wt%, and still more preferably from 0.1 to 0.3 wt% of the composition of which they are a part.

It should be understood that the hydrocolloid referred to immediately above is in addition to the components introduced as part of the primary cell wall material.

A further suitable additive comprises a particulate silica or clay, such as an amorphous silica, e.g. a fumed silica. It is particularly desirable to employ such a fumed (sometimes called pyrogenic) silica which has been hydrophobically treated. Such materials are commercially available under the name hydrophobic silica. Hydrophobic silicas are obtained by chemically bonding a hydrophbic substituent such as especially a siloxane group onto the surface of the silica, possibly following an intermediate treatment in which the surface of the silica has been rendered hydrophilic. Suitable reactants to generate a hydrophobic substituent include halosilanes and in particular chlorosilanes and methylated silazanes such as hexamethyldisilazane. It is particularly desirable to employ a silica that is capable of thickening an oil such as a plant oil. Suitable clays may include montmorillonite or hectorite.

Silica or clay is often from 0.2 to 2.0 wt%, more preferably from 0.3 to 1.5 wt%, even more preferably from 0.5 to 1.0 wt%, and still more preferably from 0.6 to 0.8 wt% of the composition of which they are a part.

*Other components in antiperspirant composition*

Other components particular to the type of composition in which the invention is used may also be included. Types of composition in which the invention may be used comprise sticks, soft solids, gels, creams, lotions and roll-ons.

The composition according to the invention delivered from a roll-on dispenser is particularly preferred. The roll-on composition may be oil-in-water or water-in-oil emulsions, preferably oil-in-water emulsion. When formulated as an emulsion, the composition according to present invention will comprise an oil such as an organic oil and/or silicone oil, particularly mineral oil, triglycerides (such as sunflower oil), volatile silicone or mixture thereof. The total oil content is often less than 10 wt% of the total composition. It is particularly suitable to employ from 1 to 5 wt% of a triglyceride oil and/or hydrocarbon oil, such as sunflower oil and/or mineral oil.

The composition may employ a non-ionic surfactant acting as an emulsifier or mixture of emulsifiers providing an HLB value in the region of 6 to 10. An especially desirable range of emulsifiers comprises a hydrophilic moiety provided by a polyalkylene oxide, particularly polyethylene oxide and a hydrophobic moiety provided by an aliphatic hydrocarbons. The hydrophobic and hydrophilic moieties can be linked via an ester, ether or ketone linkage. It is particularly suitable to employ emulsifiers such as Tween and/or steareth.

Roll-on compositions preferably comprise a sensory modifier, for example finely divided clay such as Aerosil 200, ex Evonik Degussa, typically at from 0.01 to 0.5 wt%. Other sensory modifiers include polyethylene (e.g. Acumist B18), talc and titanium dioxide. Additional deodorant actives may also be included. When employed, the level of incorporation is preferably 0.01%-3% and more preferably from 0.03%-0.5% by weight. Preferred deodorant actives are those that are more efficacious than simple alcohols such as ethanol. Examples include quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals; 2',4,4'-trichloro,2-hydroxydiphenyl ether (triclosan); and 3,7,11-trimethyldodeca-2,6,10-trienol (farnesol).In many embodiments of the invention, fragrance is a desirable additional component. Suitable materials include conventional perfumes, such as perfume oils and also include so-called deo-perfumes, as described in EP 545,556, for example. Levels of incorporation are preferably up to 5% by weight, particularly from 0.1% to 3.5% by weight, and especially from 0.5% to 2.5% by weight. The fragrance may also be added in an encapsulated form, release being triggered post-application by hydrolysis or shear on the surface of the human body.

Further additional components that may also be included are colourants and preservatives at a conventional level, for example C1-C3 alkyl parabens.In some compositions water miscible emollients such as polyethylenglycols (eg PEG 400) can be included, typically at levels up to 2 wt% of the total composition.

*Product dispenser*

The aqueous composition according to present invention can be in the form of a roll-on, lotion, cream or gel, preferably a roll-on, contained within a dispenser that allows flow of the composition onto the surface of the human body. A roll-on dispenser typically comprises a housing having an opening through which the contents flow out under gravity or under mild pressure exerted by grasping the dispenser or are conveyed out by a flow regulator. The housing defines the opening within which a rotatable ball or roller is seated, dimensioned such that in operation there is a narrow passage between the ball or roller and its housing connecting the interior of the dispenser with its exterior. Suitable dispensers for roll-ons are exemplified in GB2272186, GB2275024, US2968826, US6511243, WO00/49908, WO00/64302, EP1618810B1 and EP1618809B1. Roll-on dispensers are very popular for dispensing liquids since the ball or roller acts as an efficient way of distributing the contents of the dispenser over skin or other application surfaces. However, if the content is too thin (e.g. a Newtonian fluid), there is a risk of undesirably dripping which can be both wasteful and potentially messy. For example, content may leak out if the dispenser has fallen on its side or if it is a so-called invert dispenser, by which is meant herein a dispenser in which the opening of the dispenser under its normal storage orientation is at the bottom of the dispenser or if an upright dispenser is stored in an invert orientation in order to ensure that its contents is employed to the last drop. The employment of primary cell wall materials in the aqueous composition can raise the elastic modulus (G') of the composition, thereby mitigating this problem.

*Method to manufacture the composition*

The method for manufacturing according to present invention comprises defibrillation of the primary cell wall material by subjecting it to sufficient mechanical energy (e.g. shear). The source of primary cell wall material preferably is non-defibrillated and commercially available, for example, as fruit and vegetable purees, Herbacel AQ Plus citrus fibre (supplied by Herbafoods). However already defibrillated material can also be suitably used.

Suitable defibrillation techniques are known in the art. The defibrillation is performed in presence of an aqueous medium. The amount of aqueous medium can vary but preferably is such that a liquid slurry is formed. The amount of aqueous medium is preferably at least 1 time, more preferably at least 5 times, even more preferably at least 10 times and most preferably at least 15-20 times the amount of primary cell wall material, wherein the latter is based on dry weight. The defibrillation can be done as a single or a succession of treatments. Defibrillation is preferably carried out using high shear treatment, pressure homogenization, cavitation, explosion, pressure increase and pressure drop treatments, colloidal milling, intensive blending, extrusion, ultrasonic treatment, extrusion, grinding and combinations thereof, more preferably by pressure homogenization treatment. Preferred pressure when using high-pressure homogenizers is at least 200bar, more preferably at least 500 bar, even more preferably at least 700bar and most preferably at least 1000bar. The equipment can be those known in the industry such as a single stage valve homogenizer, an ultra high pressure (UHP) homogenizer or a microfluidizer.

The method of manufacturing the composition according to present invention comprises steps of: i) defibrillation of primary cell wall material in aqueous medium; ii) addition/dissolution of the water soluble antiperspirant active; iii) addition of other ingredients. Alternatively, the method may comprise steps of: i) in aqueous medium, mixing primary cell wall material and one or more of the other ingredients other than antiperspirant active; ii) defibrillation of the mixture; iii) addition of the remaining ingredients including the antiperspirant active. Still alternatively, the method may comprise steps of: i) in aqueous medium, mixing primary cell wall material, antiperspirant active and optionally, at least one of the other ingredients in the composition; ii) defibrillation of the mixture; iii) addition of the remaining ingredients. It is desirable to add the water soluble antiperspirant active after defibrillation of the primary cell wall material. This process can potentially avoid generating excessive heat from the exothermic dissolution of antiperspirant active during defibrillation. If the composition is an emulsion, it is desirable to defibrillate oil and primary cell wall material together, optionally with one or more ingredients in the antiperspirant composition other than the antiperspirant active. This eases the manufacture by avoiding adding oil as a separate component in later stage of the process.

The invention may be illustrated by the following non-limiting examples.

## EXAMPLES

The citrus fibre (powder) was obtained from Herbafood Ingredients GmBH with tradename Citrus Fibre AQ+N. Sugar Cane Fibre (powder) was obtained from Watson InC with tradename UltraCel™. Tomato fibre (disperson) was obtained from Agraz, SAU with tradename 'Brix'. Mineral oil and Tween 60® were obtained from Sonnebone and Sigma-Aldrich respectively.

The composition of the primary cell wall materials is shown in Table 3 below.

**Table 3:** composition of primary cell wall material

| Source | Cellulose microfibrils (%) | Hemicelluloses (%) | Pectin (%) | Other (moisture) (%) |
|---|---|---|---|---|
| Citrus fiber (powder) | 50 | 15-20 | 10-15 | ~7 (moisture) |
| Tomato fiber (dispersion) | 50 | 15-20 | 10-15 | ND |
| Sugar cane fiber (powder) | 95 | 0 | 0 | $8 \pm 3$ (moisture) |
| ND= not determined | | | | |

The defibrillation of citrus fibre is here below given as an example.

### Defibrillation of microfibrillated cellulose from citrus fibre

The citrus fibre was dispersed in water and allowed to swell amid slow agitation using silverson high-speed mixer. After swelling, silverson was run at 5000 RPM for 5 - 10 minutes. The effect of the difference between 5 and 10 minutes of stirring on the final structure was completely overruled by the high shear in the next step. The dispersion is then processed using a Microfluidizer ™ (MF) high pressure homogeniser equipped with a Z-chamber (87um) in one pass at pressure of 1000-1200bar. Alternatively, a Panda high pressure homogeniser (HPH) operating at 260-340 bar can be used for the final defibrillation instead of a MF with Z-chamber.

In the example where Tween 60 and mineral oil were used, those two ingredients were added to the dispersion immediately after the running of silverson at 5000rpm for 5-10 minutes.

### Preparation of the antiperspirant composition

Aluminium chlorohydrate (ACH) was obtained from Summit Research Labs, Inc. Aluminium chloride ($AlCl_3$) anhydrous was obtained from Fluka. The salts were dissolved in demineralized water to obtain 50% solution, then added to the prepared, defibrillated fibre dispersion in such a way that the final concentration of the fibre ranged from 0.1-1.0%. The final concentration of Aluminum salt was 20% for formulations in Table 4 and 5. The final concentration of aluminium salt was 10% for formulations in Table 6. C1, C2 and C3 are prepared not according to the invention and all other samples are prepared according to the invention.

**Table 4 :** Antiperspirant composition (i)

| | C1 | C2 | EX1 | EX2 | EX3 | EX4 | EX5.1 | EX6 |
|---|---|---|---|---|---|---|---|---|
| Citrus fibre | | | 0.1 | 0.2 | 0.3 | 0.4 | 0.5 | 1.0 |
| $AlCl_3$ | 20 | | 20.0 | 20.0 | 20.0 | 20.0 | 20.0 | 20.0 |
| ACH | | 20 | | | | | | |
| Demin. water | To 100% | | | | | | | |

**Table 5** : Antiperspirant composition (ii)

|  | **EX5.2** | **EX5.3** | **EX7** | **EX8** |
|---|---|---|---|---|
| Tomato fibre |  | 0.5 |  | 0.5 |
| Sugar cane fibre | 0.5 |  | 0.5 |  |
| $AlCl_3$ | 20.0 | 20.0 |  |  |
| ACH |  |  | 20.0 | 20.0 |
| Demin. water | To 100% |  |  |  |

**Table 6:** Antiperspirant composition (iii)

|  | **C3** | **EX9** |
|---|---|---|
| Citrus fibre |  | 0.4 |
| Tween 60® | 0.5 | 0.5 |
| Mineral oil | 5 | 5 |
| $AlCl_3$ | 10.0 | 10.0 |
| Demin. water | To 100% |  |

### Rheology characterisation

All measurements were carried out at 20°C.Viscoelastic property was determined by using a controlled stress rheometer, Physica MCR300 or MCR301 from Anton Paar. The rheometer was equipped with geometry of profiled concentric cylinders (cup diameter 30mm). A programme of oscillatory stress sweep was used. Elastic modulus G' and viscous modulus G" as a function of stress from 0.01 to1000Pa respectively a strain from 0.01 to 1000% at a frequency of 1 Hz were measured during ten minutes. This range of stresses respectively strains includes the value at which the structure of the viscoelastic material breaks and the material starts to flow. Herein, G'/G" at the linear plateau (before the breaking of the structure) is reported. Newtonian solution capable of dripping has G'/G" smaller than 1. In contrast, a thickened-up dispersion with a percolated network to ameliorate the problem has a G'/G" ratio of at least 1. A programme of flow measurement was used to determine the viscosity of the composition in respective of shear rate. A ten-minute continuous ramp of shear rate was used from 0 s$^{-1}$ to 1000 s$^{-1}$, followed by an additional step where the shear rate was continuously decreased in another ten minutes from 1000 s$^{-1}$ to 0 s$^{-1}$. In example of a roll-on antiperspirant product, the composition must have a high viscosity at zero or low shear to prevent dripping from the packaging. At moderate shear, the composition should have a low viscosity that enables it to flow out of the pack for easy application by consumers. Herein, viscosity of the composition at two shear rates (1.0 s$^{-1}$ and 49.2 s$^{-1}$) is reported.

### Viscoelastic modulus (G'/G") of the composition

**Table 7:** G'/G" for antiperspirant compositions (i)

| Ref | Fibre concentration (wt%) | G'/G" | | |
|---|---|---|---|---|
|  |  | Citrus fiber | Sugar Cane fiber | Tomato puree fiber |
| C1 | 0 | <1 | | 15 |
| EX1 | 0.1 | 2.4 |  |  |
| EX2 | 0.2 | 3.1 |  |  |
| EX3 | 0.3 | 3.7 |  |  |
| EX4 | 0.4 | 3.8 |  |  |
| EX5.1-5.3 | 0.5 | 4.2 (EX5.1) | 7.7 (EX 5.2) | 6.2 (EX 5.3) |

(continued)

| Ref | Fibre concentration (wt%) | G'/G" | | |
| --- | --- | --- | --- | --- |
| | | Citrus fiber | Sugar Cane fiber | Tomato puree fiber |
| EX6 | 1.0 | 4.7 | | |

**Table 8** : G'/G" for antiperspirant compositions (ii)

| Ref | Fibre concentration (wt%) | G'/G" | | |
| --- | --- | --- | --- | --- |
| | | Citrus fiber | Sugar Cane fiber | Tomato puree fiber |
| C2 | 0 | <1 | | |
| EX7 | 0.5 | | 8.0 | |
| EX8 | 0.5 | | | 6.2 |

**Table 9:** G'/G" for antiperspirant compositions (iii)

| Ref | Fibre concentration (wt%) | G'/G" |
| --- | --- | --- |
| C3 | 0 | <1 |
| EX9 | 0.4 | 5.0 |

When compared in terms of product rheology, it is found that EX1-EX9 according to the invention all showed superior G'/G" values to C1-C3 (G'/G"<1) which are not according to the invention. In the final stage of defibrillation, all fibres are defibrillated by MF at approximately 1100 bar.

*Viscosity of the compositions*

**Table 10:** Viscosity for antiperspirant compositions (i)

| | EX1 | EX2 | EX3 | EX4 | EX5.1 | EX6 |
| --- | --- | --- | --- | --- | --- | --- |
| Citrus fibre% | 0.1 | 0.2 | 0.3 | 0.4 | 0.5 | 1 |
| Viscosity/Pa s (1.0 s$^{-1}$) | 0.562 | 1.44 | 2.88 | 4.24 | 8.1 | 39.5 |
| Viscosity/Pa s (49.2 s$^{-1,}$up -flow) | 0.0345 | 0.0748 | 0.134 | 0.192 | 0.351 | 1.29 |

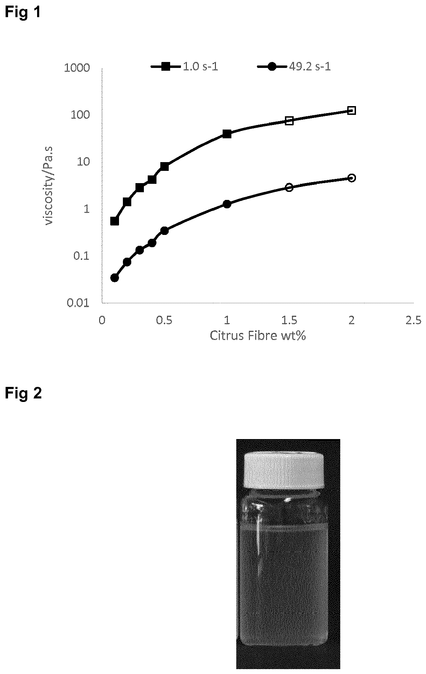

In Table 10, all samples contain 20 wt% AlCl$_3$ and the citrus fibre is defibrillated by MF at approximately 1100bar. Figure 1 graphically illustrates the viscosity of antiperspirant compositions (i) with the hollow data estimated based upon extrapolation. It can be seen that at low shear rates the viscosity of the composition progressively increases along with the increase of citrus fibre and the effect is most pronounced around 0.5% citrus fibre. This benefit is less pronounced at fibre concentration >1 wt% (by extrapolating data). The composition also shows shear-thinning property at shear rate of 49.2 s$^{-1}$, which enables them to flow out of the corresponding packaging upon use.

*Elastic modulus G' of different defibrillation process*

**Table 11:** Elastic modulus G' for antiperspirant composition (i)

| | 0.2wt % citrus fibre (EX2) | 0.5 wt% citrus fibre (EX5.1) | 1.0 wt% citrus fibre (EX6) |
| --- | --- | --- | --- |
| MF, -1100 bar | 3.9 | 49 | 300 |

(continued)

|  | 0.2wt % citrus fibre (EX2) | 0.5 wt% citrus fibre (EX5.1) | 1.0 wt% citrus fibre (EX6) |
|---|---|---|---|
| HPH, -300 bar | 0.18 * | 4.3 | 93 |
| G'(MF) / G'(HPH) | 21.7 | 11.4 | 3.2 |
| *Sample is short-lived with sedimentation observed quickly. | | | |

All samples contain 20 wt% $AlCl_3$. The samples contain different levels of citrus fibres and the fibres are defibrillated by MF with Z-chamber at about 1100 bar and by HPH at about 300 bar respectively. Table 11 gives elastic modulus G' (at linear plateau). Overall, fibres defibrillated at shear above 1000bar is preferred.

*Storage and Appearance*

Further examination of EX1-EX5.1 showed the gel structure is at least stable for 60 days at ambient, during which there is no observation of sedimentation or syneresis.

Additionally, EX1-3 give almost transparent appearance. Figure 2 is a picture showing the appearance of composition EX1. It may be due to the refractive index match between the Aluminium salt and citrus fibre. It should be understood that it is within the skilled person's capacity to manipulate the concentration within the range of interests to achieve the aesthetic clarity of the composition.

**Claims**

1. An aqueous antiperspirant composition comprising:

    i) at least 2 wt% of water soluble, antiperspirant active comprising aluminium ions and chloride ions, wherein the water soluble antiperspirant active is an astringent antiperspirant salt selected from an aluminium or an aluminium-zirconium chlorohydrate salt or complex, and
    ii) from 0.1 to 4.0 wt% of defibrillated primary cell wall material,

    wherein at least 50 wt% of the total material is sourced from plant parenchymal tissue and wherein the defibrillated primary cell wall material comprises at least 50 wt% of cellulose microfibrils, based upon the total dry weight of the defibrillated primary cell wall material.

2. A composition according to claim 1, wherein the defibrillated primary cell wall material has an average crystallinity of less than 50%.

3. A composition according to any of the preceding claims, wherein at least 80% by weight of the defibrillated primary cell wall material has a diameter of less than 50nm.

4. A composition according to any of the preceding claims, wherein the defibrillated primary cell wall material is sourced from parenchymal tissue selected from citrus fiber, tomato fiber, sugar cane fiber and mixtures thererof.

5. A composition according to any of the preceding claims, wherein the water soluble antiperspirant active is present at from 4 to 25% by weight, preferably from 10 to 20% by weight.

6. A composition according to any of the preceding claims, wherein the water soluble antiperspirant salt is aluminium sesquichlorohydrate.

7. A composition according to any of the preceding claims, comprising an oil phase, wherein the composition is in the form of oil-in-water emulsion

8. A composition according to any of the preceding claims, in the form of a roll-on, lotion, cream or gel.

9. A product comprising a composition according to any of the preceding claims, contained within a dispenser that allows flow of the composition onto the surface of the human body.

10. A product according to claim 9 that is a roll-on composition contained within a roll-on dispenser.

11. A manufacturing process for the composition according to any of claims 1 to 8, comprising the defibrillation in an aqueous medium of primary cell wall material sourced from plant parenchymal tissue, wherein during or after the defibrillation a water soluble antiperspirant active is mixed in.

12. A manufacturing process according to claim 11, wherein the defibrillation comprises a high-shear treatment step using a high pressure homogenizer at the pressure of at least 200bar, preferably at least 500bar, most preferably at least 1000bar.

13. A method of reducing perspiration from human skin in which an antiperspirant composition according to claim 1-8 is applied to the axillary skin.

14. A method of thickening an aqueous antiperspirant composition comprising at least 2 wt.% of water soluble antiperspirant active comprising aluminium ions and chloride ions, wherein the water soluble antiperspirant active is an astringent antiperspirant salt selected from an aluminium or an aluminium-zirconium chlorohydrate salt or complex said method comprising the addition of from 0.1 to 4.0 wt.% of defibrillated primary cell wall material wherein at least 50 wt% of the total material is sourced from plant parenchymal tissue, and wherein the defibrillated primary cell wall material comprises at least 50 wt% of cellulose microfibrils, based upon the total dry weight of the defibrillated primary cell wall material.


**Patentansprüche**

1. Wässrige schweißhemmende Zusammensetzung, umfassend:

   i) mindestens 2 Gew.-% wasserlöslichen, schweißhemmenden Wirkstoff, umfassend Aluminiumionen und Chloridionen, wo der wasserlösliche, schweißhemmende Wirkstoff ein astringentes schweißhemmendes Salz ist, ausgewählt aus einem Aluminium- oder einem Aluminium-Zirkonium-Chlorhydrat-Salz oder -Komplex, und
   ii) 0,1 bis 4,0 Gew.-% defibrilliertes primäres Zellwandmaterial,

   wobei mindestens 50 Gew.-% des gesamten Materials von Pflanzenparenchymgewebe stammen und wobei das defibrillierte primäre Zellwandmaterial mindestens 50 Gew.-% Cellulosemikrofibrillen, bezogen auf das Gesamttrockengewicht des defibrillierten primären Zellwandmaterials, umfasst.

2. Zusammensetzung nach Anspruch 1, wobei das defibrillierte primäre Zellwandmaterial eine durchschnittliche Kristallinität von weniger als 50% aufweist.

3. Zusammensetzung nach einem der vorhergehenden Ansprüche, wobei mindestens 80 Gewichts-% des defibrillierten primären Zellwandmaterials einen Durchmesser von weniger als 50 nm aufweisen.

4. Zusammensetzung nach einem der vorhergehenden Ansprüche, wobei das defibrillierte primäre Zellwandmaterial von Parenchymgewebe, ausgewählt aus Citrusfaser, Tomatenfaser, Zuckerrohrfaser und Mischungen davon, stammt.

5. Zusammensetzung nach einem der vorhergehenden Ansprüche, wobei der wasserlösliche schweißhemmende Wirkstoff mit 4 bis 25 Gewichts-%, vorzugsweise 10 bis 20 Gewichts-%, vorhanden ist.

6. Zusammensetzung nach einem der vorhergehenden Ansprüche, wobei das wasserlösliche schweißhemmende Salz Aluminiumsesquichlorhydrat ist.

7. Zusammensetzung nach einem der vorhergehenden Ansprüche, umfassend eine Ölphase, wobei die Zusammensetzung in Form einer Öl-in-Wasser-Emulsion vorliegt.

8. Zusammensetzung nach einem der vorhergehenden Ansprüche in Form eines Roll-on, einer Lotion, einer Creme oder eines Gels.

9. Produkt, umfassend eine Zusammensetzung nach einem der vorhergehenden Ansprüche, enthalten in einem Spender, der das Fließen der Zusammensetzung auf die Oberfläche des menschlichen Körpers ermöglicht.

**10.** Produkt nach Anspruch 9, das eine Roll-on-Zusammensetzung ist, die in einem Roll-on-Spender enthalten ist.

**11.** Verfahren zur Herstellung der Zusammensetzung nach einem der Ansprüche 1 bis 8, umfassend das Defibrillieren von primärem Zellwandmaterial, das von Pflanzenparenchymgewebe stammt, in einem wässrigen Medium, wobei während oder nach dem Defibrillieren ein wasserlöslicher schweißhemmender Wirkstoff eingemischt wird.

**12.** Verfahren zur Herstellung nach Anspruch 11, wobei das Defibrillieren einen Behandlungsschritt mit hoher Scherung unter Verwendung eines Hochdruckhomogenisators bei einem Druck von mindestens 200 bar, vorzugsweise mindestens 500 bar, höchst bevorzugt von mindestens 1000 bar, umfasst.

**13.** Verfahren zur Verringerung der Transpiration menschlicher Haut, bei dem eine schweißhemmende Zusammensetzung nach den Ansprüchen 1-8 auf die Achselhaut aufgetragen wird.

**14.** Verfahren zum Verdicken einer wässrigen schweißhemmenden Zusammensetzung, umfassend mindestens 2 Gew.-% wasserlöslichen schweißhemmenden Wirkstoff, umfassend Aluminiumionen und Chlorionen, wobei der wasserlösliche schweißhemmende Wirkstoff ein astringentes schweißhemmendes Salz ist, ausgewählt aus einem Aluminium- oder einem Aluminium-Zirkonium-Chlorhydrat-Salz oder -Komplex, wobei das Verfahren die Zugabe von 0,1 bis 4,0 Gew.-% defibrilliertes primäres Zellwandmaterial umfasst, wobei mindestens 50 Gew.-% des Gesamtmaterials von Pflanzenparenchymgewebe stammen und wobei das defibrillierte primäre Zellwandmaterial mindestens 50 Gew.-% Cellulosemikrofibrillen, bezogen auf das Gesamttrockengewicht des defibrillierten primären Zellwandmaterials umfasst.

## Revendications

**1.** Composition aqueuse d'antiperspirant comprenant :

i) au moins 2 % en masse d'actif d'antiperspirant, soluble dans l'eau, comprenant des ions aluminium et ions chlore, où l'actif d'antiperspirant soluble dans l'eau est un sel d'antiperspirant astringent choisi parmi un aluminium ou un sel ou complexe de chlorohydrate d'aluminium-zirconium, et
ii) de 0,1 à 4,0 % en masse de matériau de paroi de cellule primaire défibrillé,

où au moins 50 % en masse de la matière totale proviennent de tissu parenchymateux de plante et où le matériau de paroi de cellule primaire défibrillé comprend au moins 50 % en masse de microfibrilles de cellulose, sur la base de la masse sèche totale du matériau de paroi de cellule primaire défibrillé.

**2.** Composition selon la revendication 1, où le matériau de paroi de cellule primaire défibrillé présente une cristallinité moyenne inférieure à 50 %.

**3.** Composition selon l'une quelconque des revendications précédentes, où au moins 80 % en masse du matériau de paroi de cellule primaire défibrillé présente un diamètre inférieur à 50 nm.

**4.** Composition selon l'une quelconque des revendications précédentes, où le matériau de paroi de cellule primaire défibrillé provient de tissu parenchymateux choisi parmi une fibre d'agrume, fibre de tomate, fibre de canne à sucre et des mélanges de celles-ci.

**5.** Composition selon l'une quelconque des revendications précédentes, où l'actif d'antiperspirant soluble dans l'eau est présent à de 4 à 25 % en masse, de préférence de 10 à 20 % en masse.

**6.** Composition selon l'une quelconque des revendications précédentes, où le sel d'antiperspirant soluble dans l'eau est le sesquichlorohydrate d'aluminium.

**7.** Composition selon l'une quelconque des revendications précédentes, comprenant une phase d'huile, où la composition est dans la forme d'une émulsion huile-dans-eau.

**8.** Composition selon l'une quelconque des revendications précédentes, dans la forme d'un roll-on, d'une lotion, d'une crème ou d'un gel.

9. Produit comprenant une composition selon l'une quelconque des revendications précédentes, contenu à l'intérieur d'un distributeur qui permet l'écoulement de la composition sur la surface du corps humain.

10. Produit selon la revendication 9 qui est une composition de roll-on contenue à l'intérieur d'un distributeur roll-on.

11. Procédé de fabrication pour la composition selon l'une quelconque des revendications 1 à 8, comprenant la défibrillation dans un milieu aqueux de matériau de paroi de cellule primaire provenant d'un tissu parenchymateux de plante, où pendant ou après la défibrillation un actif d'antiperspirant soluble dans l'eau est mélangé.

12. Procédé de fabrication selon la revendication 11, où la défibrillation comprend une étape de traitement à cisaillement élevé utilisant un dispositif d'homogénéisation haute pression à la pression d'au moins 200 bars, de préférence d'au moins 500 bars, encore mieux d'au moins 1 000 bars.

13. Procédé de réduction de la transpiration de la peau humaine dans lequel une composition d'antiperspirant selon la revendication 1-8 est appliquée à la peau axillaire.

14. Procédé d'épaississement d'une composition aqueuse d'antiperspirant comprenant au moins 2 % en masse d'actif d'antiperspirant soluble dans l'eau comprenant des ions aluminium et ions chlore, dans lequel l'actif d'antiperspirant soluble dans l'eau est un sel d'antiperspirant astringent choisi parmi un aluminium ou un sel ou complexe de chlorohydrate d'aluminium-zirconium ledit procédé comprenant l'addition de 0,1 à 4,0 % en masse de matériau de paroi de cellule primaire défibrillé où au moins 50 % en masse du matériau total proviennent de tissu parenchymateux de plante, et où le matériau de paroi de cellule primaire défibrillé comprend au moins 50 % en masse de microfibrilles de cellulose, sur la base de la masse sèche totale du matériau de paroi de cellule primaire défibrillé.

**Fig 1**

**Fig 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 12084441 A
- WO 12084421 A
- WO 14082835 A
- WO 14082951 A
- WO 15006635 A
- WO 15006634 A
- WO 13160022 A
- WO 12052306 A
- US 2015210957 A
- WO 13160024 A
- US 2012040003 A
- WO 13107771 A
- EP 0006739 A
- WO 2014187685 A
- US 3792068 A
- US 20010004869 A1
- EP 545556 A
- GB 2272186 A
- GB 2275024 A
- US 2968826 A
- US 6511243 B
- WO 0049908 A
- WO 0064302 A
- EP 1618810 B1
- EP 1618809 B1

### Non-patent literature cited in the description

- **M.A JOSLYN ; GUIDO DE LUCA.** *J.Colloid Sci.,* 1957, vol. 12 (1), 108-130
- Deodorant Ingredients. **S.A.MAKIN ; M.R.LOWRY.** Antiperspirants and Deodorants. Marcel Dekker, 1999